# EUROPÄISCHE PATENTSCHRIFT

(11) **EP 2 355 693 B1**
(45) Veröffentlichungstag und Bekanntmachung des Hinweises auf die Patenterteilung: **19.12.2012**
(21) Anmeldenummer: 09778675.0
(22) Anmeldetag: 23.09.2009
(51) Int. Cl.: A61B 5/00

(54) **VORRICHTUNG UND VERFAHREN ZUM ERFASSEN EINES VITALPARAMETERS**
APPARATUS AND METHOD FOR RECORDING A VITAL PARAMETER
DISPOSITIF ET PROCÉDÉ POUR DÉTECTER UN PARAMÈTRE VITAL

(30) Priorität: 07.10.2008 DE 102008050638; 06.11.2008 DE 102008056251
(43) Veröffentlichungstag der Anmeldung: 17.08.2011
(73) Patentinhaber: Fraunhofer-Gesellschaft zur Förderung der angewandten Forschung e.V., 80686 München (DE); ZF Friedrichshafen AG, 88038 Friedrichshafen (DE)
(72) Erfinder: COURONNÉ, Robert, 91058 Erlangen (DE); CIANCITTO, Fabio, 91056 Erlangen (DE); ERSHOV, Sergey, 91054 Erlangen (DE); WEIGAND, Christian, 90556 Cadolzburg (DE); GASSMANN, Hans-Josef, 31715 Meerbeck (DE); HAEVESCHER, Rainer, 32351 Stemwede (DE)
(74) Vertreter: Hersina, Günter
(86) Internationale Anmeldenummer: PCT/EP2009/006875
(87) Internationale Veröffentlichungsnummer: WO 2010/040451

(56) Entgegenhaltungen:
- US-A- 5 792 052
- US-A1- 2003 036 685

## Beschreibung

Die vorliegende Erfindung bezieht sich auf Vorrichtungen und Verfahren zum Erfassen eines Vitalparameters und beispielsweise auf die Ansteuerung dazu verwendeter optoelektronischer Sensoranordnungen.

Die Verfahren der optischen Plethysmographie und Pulsoxymetrie sind Methoden zur nicht-invasiven Ermittlung von Vitalparametern eines Lebewesens, z. B der Pulsrate, der Pulsratenvariabilität und der arteriellen Sauerstoffsättigung über die Messung einer Lichtabsorption bzw. einer Lichtremission im Gewebe des Lebewesens.

Typischerweise werden die Sauerstoffsättigungswerte bzw. SpO₂-Werte über einen optischen Sensor am Finger, Zeh oder Ohrläppchen abgenommen, wobei die Messung mit einem Clipsensor oder Klebesensor erfolgt.

Die Anwendung der optischen Plethysmographie und Pulsoxymetrie zum Erfassen solcher Vitalparameter kann jedoch auch unter Automobilbedingungen, d. h. in einem Automobil bzw. Kraftfahrzeug durchgeführt werden, z. B. durch Integration der optischen Sensoren in Bedienelemente des Kraftfahrzeugs. Dies ermöglicht eine Erfassung der Vitalparameter mit geringer Beeinträchtigung des Fahrers. Eine mögliche Implementierung ist die Integration des optischen Sensors in einen Schaltknauf des Kraftfahrzeugs.

Die Sensoren bestehen typischerweise aus zwei Lichtquellen, z. B. einer roten Dioden und einer infraroten Diode und einem Photosensor bzw. Photodiode. Sobald der optische Sensor eingeschaltet ist, leuchten die beiden Dioden. Die infrarote Diode strahlt im nicht-sichtbaren Bereich des elektromagnetischen Spektrums, im Gegensatz dazu strahlt die rote Diode im sichtbaren Bereich des elektromagnetischen Spektrums. Dies kann einerseits den Fahrer ablenken und andererseits das Lichtdesign des Automobilinnenraums negativ beeinflussen, z. B. wenn die vorherrschende Farbe grün oder blau ist.

Die US-Veröffentlichung US 5,792,052 beschreibt einen Fingerclip-Pulsoximeter, das mittels zwei LED und einem Photosensor den Puls und die Sauerstoffsättigung eines Menschen messen kann. Die Messung kann durch ein Transmissionsverfahren oder ein Reflexionsverfahren durchgeführt werden. Das Pulsoximeter ist ferner ausgebildet, automatisch zu erkennen, wenn ein Finger in das Fingerclip-Pulsoximeter eingeführt ist. Dafür wird die Strahlung eines oder beider der LED an dem Photosensor ausgewertet, wobei bei einer Transmissionsmessung ein Finger dann erkannt wird, wenn die Lichtmessung einen signifikanten Abfall der Lichtintensität misst, und bei einer Reflexionsmessung, wenn ein signifikantes Ansteigen des Lichtes gemessen wird. Ferner beschreibt die Veröffentlichung ein Ausschalten, wenn bei einer Transmissionsmessung das gemessene Licht nahe eines maximalen Referenzwertes ist und dieser für beispielsweise 10 Sekunden anhält. Die Veröffentlichung US 2003/0036685 A1 beschreibt ein physiologisches Signalüberwachungssystem mit einem Photoplethysmographie-Sensor (PPG-Sensor), der eine rote LED und eine infrarote LED sowie zwei jeweils diesen LED zugeordneten Photosensoren aufweist, um beispielsweise die Sauerstoffsättigung eines Patienten zu messen. Dabei kann die Anwesenheit oder Abwesenheit eines Fingers an dem PPG-Sensor durch regelmäßiges Anschalten einer LED und dem Erkennen, ob die entsprechende Photodiode reflektiertes Licht empfängt, durchgeführt werden.

### Zusammenfassung

Ein Ausführungsbeispiel der vorliegenden Erfindung schafft eine Vorrichtung zum Erfassen eines Vitalparameters mit folgenden Merkmalen: einer optoelektronischen Sensoranordnung zum Erfassen des Vitalparameters mittels Lichtremission, wobei die optoelektronische Sensoranordnung eine erste Lichtquelle zum Erzeugen eines Lichts im sichtbaren Wellenlängenbereich aufweist, eine zweite Lichtquelle zum Erzeugen eines Lichts in einem nicht-sichtbaren Wellenlängenbereich aufweist und ein lichtempfindliches Element; und einer Steuereinrichtung, die ausgebildet ist, um die zweite Lichtquelle in zeitlichen Abständen anzuschalten, eine Auswertung des von dem lichtempfindlichen Element empfangenen Lichts in dem unsichtbaren Wellenlängenbereich der zweiten Lichtquelle dahin gehend durchzuführen, ob ein Finger an der optoelektronischen Sensoranordnung anliegt, und die erste Lichtquelle anzuschalten, sobald die Auswertung ergibt, dass ein Finger an der optoelektronischen Sensoranordnung anliegt, wobei die Steuereinrichtung (1410) ausgebildet ist, die Aus- wertung über einen Zeitverlauf des von dem lichtemp- findlichen Element (1330) empfangenen Lichts in dem unsichtbaren Wellenlängenbereich der zweiten Licht- quelle (1320) durchzuführen, um einen Pulsparameter zu erkennen, wenn ein Finger an der optoelektronischen Sensoranordnung (1130) anliegt, und die erste Licht- quelle (1310) anzuschalten, wenn eine Pulswelle erkannt wird.

Ein weiteres Ausführungsbeispiel der vorliegenden Erfindung schafft ein Verfahren zum Erfassen eines Vitalparameters mittels einer optoelektronischen Sensoranordnung zum Erfassen des Vitalparameters mittels Lichtremission, wobei die optoelektronische Sensoranordnung eine erste Lichtquelle zum Erzeugen eines Lichts im sichtbaren Wellenlängenbereich aufweist, eine zweite Lichtquelle zum Erzeugen eines Lichts in einem nicht-sichtbaren Wellenlängenbereich aufweist und ein lichtempfindliches Element aufweist, und wobei das Verfahren folgende Schritte aufweist: Einschalten der zweiten Lichtquelle in zeitlichen Abständen; Auswerten des von dem lichtempfindlichen Element empfangenen Lichts in dem unsichtbaren Wellenlängenbereich der zweiten Lichtquelle dahin gehend, ob ein Finger an der optoelektronischen Sensoranordnung anliegt; und Anschalten der ersten Lichtquelle, sobald die Auswertung ergibt, dass ein Finger an der optoelektronischen Sensoranordnung anliegt, wobei das Auswerten über einen Zeitverlauf des von dem lichtempfindlichen Element (1330) empfangenen Lichts in dem unsichtbaren Wellenlängenbereich der zweiten Lichtquelle (1320) durchgeführt wird, um einen Pulsparameter zu erkennen, wenn ein Finger an der optoelektronischen Sensoranordnung (1130) anliegt, und die erste Lichtquelle (1310) angeschaltet wird, wenn eine Pulswelle erkannt wird.

Wie zuvor dargelegt, kann der Dauerbetrieb des aktiven Sensormoduls, welches zwei lichtemittierende Dioden umfasst, bei nicht aufliegendem Finger zur Beeinträchtigung einerseits des Fahrers sowie andererseits der Lichtgestaltung im Fahrzeuginnenraum führen. Diese Effekte treten insbesondere in Abend- und Nachtstunden störend in Erscheinung. Ausführungsbeispiele der vorliegenden Erfindung bewirken ein Abschalten der das sichtbare Licht emittierenden Diode zu den Zeiten, in denen sich kein Finger an der optoelektronischen Sensoranordnung bzw. an dem aktiven Sensormodul befindet. Damit kann wirksam die störende Beeinträchtigung von Fahrer und Lichtgestaltung vermieden werden.

Ferner wird durch das Abschalten des das sichtbare Licht emittierenden Diode der gesamte Energieverbrauch des Sensors verringert.

In der vorliegenden Anmeldung werden für den Begriff "optoelektronische Sensoranordnung" allgemein auch Sensor oder Sensormodul verwendet. Ferner bildet eine rote Diode ein Ausführungsbeispiel für die erste Lichtquelle, die ausgebildet ist, um ein Licht im sichtbaren Wellenlängenbereich bzw. im sichtbaren elektromagnetischen Spektrum zu erzeugen, eine infrarote Diode ein Ausführungsbeispiel für die zweite Lichtquelle, die ausgebildet ist, ein Licht im nicht-sichtbaren Wellenlängenbereich bzw. im nicht-sichtbaren elektromagnetischen Spektrum zu erzeugen, und ein Photosensor bzw. eine Photodiode ein Ausführungsbeispiel für ein lichtempfindliches Element. Ferner ist der Begriff "Vitalparameter" ein Oberbegriff für medizinische Parameter wie z. B. der Blutsauerstoffsättigung, dem Puls, dem Elektrokardiogramm (EKG), der Temperatur, der Muskelspannungen, des Blutzuckergehalts oder des Blutdrucks. Dabei kann in Bezug auf den "Puls" noch zwischen den Pulsparametern bzw. Pulsinformationen Pulswelle, Pulsfrequenz, Pulsamplitude, Form der Pulswelle und/oder der Geschwindigkeit der Ausbreitung der Pulswelle unterschieden werden.

Ausführungsbeispiele der Erfindung betreffen somit auch eine technische Lösung zur Ansteuerung der Einschaltanordnung eines optischen Sensors zur Detektion einer Pulswelle, Pulsrate, einer Pulsratenvariabilität und einer Sauerstoffsättigung des Blutes eines Fahrers eines Kraftfahrzeugs.

Anwendungsgebiete der Ausführungsbeispiele liegen z. B. im Bereich der präventiven, überwachenden und begleitenden Medizin für den Einsatz im Fahrzeug, z. B. als Fahrerassistenzsystem.

### Kurzbeschreibung der Figuren

Ausführungsbeispiele der vorliegenden Erfindung werden nachfolgend unter Bezugnahme auf beiliegende Zeichnungen näher erläutert.
- Fig. 1: zeigt ein Blockdiagramm eines Ausführungsbeispiels einer Vorrichtung zum Erfassen eines Vitalparameters mit einer optoelektronischen Sensoranordnung und einer Steuereinrichtung.
- Fig. 2: zeigt ein Flussdiagramm eines Ausführungsbeispiels eines Verfahrens zum Erfassen eines Vitalparameters.
- Fig. 3A bis 3D: zeigen beispielhafte Verläufe von Originalsignalen und daraus mittels morphologischer, nichtlinearer Operatoren erzeugten Signalen.

In der Beschreibung werden für Objekte und Funktionseinheiten, die gleiche oder ähnliche funktionelle Eigenschaften aufweisen, gleiche Bezugszeichen verwendet, wobei eine wiederholte Beschreibung dieser Objekte und Funktionseinheiten weggelassen wird.

### Detaillierte Beschreibung

Wie zuvor erläutert, kann eine dauerhafte Aktivierung der ersten Lichtquelle, die ein Licht im sichtbaren Wellenlängenbereich erzeugt, insbesondere in den Abend- und Nachtstunden den Fahrer stören und/oder die Lichtgestaltung des Kraftfahrzeuginnenraums stören.

Zum besseren Verständnis der vorliegenden Erfindung wird vorab als Beispiel ein Sensor zum Erfassen eines Vitalparameters beschrieben, der eine rote und eine infrarote Diode als Lichtquellen aufweist, und einen Photosensor bzw. eine Photodiode als lichtempfindliches Element. Die Lichtquellen und der Photosensor liegen beispielsweise in derselben Ebene und sind nah zueinander angeordnet. Die Lichtquellen strahlen ihr Licht in das Gewebe des Fingers und der Photosensor misst die reflektierten, remittierten Anteile des Lichtfeldes. Das Gewebe ist ständig durchblutet, wobei die Durchblutung nicht konstant ist, sondern zeitlich in Form der Pulswellen variiert. Das Blut besitzt die Eigenschaft, das Licht in verschiedenen Wellenlängen unterschiedlich zu absorbieren. Der Grad der Absorption des Lichtfeldes bzw. der reflektierte Anteil des Lichtfeldes im Zeitverlauf liefert die Information über die Pulswellen im Gewebe. Dabei ist für das Erfassen von Eigenschaften der Pulswelle (z. B. die Form, die Geschwindigkeit der Ausbreitung, die Pulsrate) die Bestimmung des Absorptionsgrades von nur einer Lichtwellenlänge, d. h. im sichtbaren oder nicht-sichtbaren Wellenlängenbereich, ausreichend. Zur Berechnung der Sauerstoffsättigung des Blutes sind jedoch zwei Lichtwellenlängen bzw. die Bestimmung des Absorptionsgrades für zwei Lichtwellenlängen notwendig.

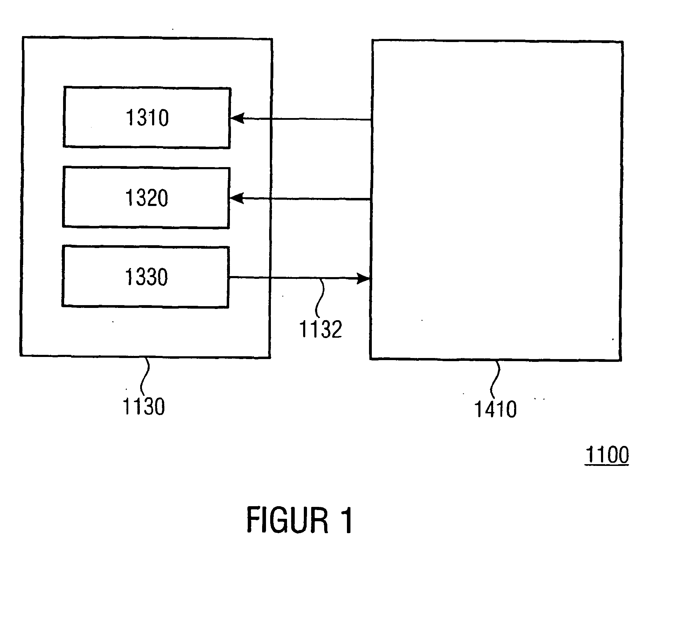

Fig. 1 zeigt ein Blockdiagramm eines Ausführungsbeispiels einer Vorrichtung 1100 zum Erfassen eines Vitalparameters, die eine optoelektronische Sensoranordnung 1130 und eine Steuereinrichtung 1410 aufweist.

Die Steuereinrichtung 1410 ist mit der optoelektronischen Sensoranordnung gekoppelt, wobei die Steuereinrichtung 1410 ausgebildet ist, um die erste Lichtquelle 1310 oder die zweite Lichtquelle 1320 zu aktivieren bzw. deaktivieren bzw. anzuschalten oder abzuschalten (siehe Pfeile von der Steuereinrichtung 1410 zu der ersten Lichtquelle und zweiten Lichtquelle 1310, 1320), und ausgebildet ist, um ein Signal von dem lichtempfindlichen Element 1330 zu erhalten, das eine Information über die Intensität bzw. Lichtstärke des empfangenen Lichts aufweist. Das Signal ist beispielsweise eine Spannung, die von dem lichtempfindlichen Element ausgegeben wird und die in einem bekannten Verhältnis von der Lichtintensität des einfallenden Lichts abhängt.

Die optoelektronische Sensoranordnung 1130 ist ausgebildet, den Vitalparameter mittels Lichtremission zu erfassen, wie dies zuvor schon beschrieben wurde. Die optoelektronische Sensoranordnung 1130 weist eine erste Lichtquelle 1310 zum Erzeugen eines Lichts in einem sichtbaren Wellenlängenbereich auf, eine zweite Lichtquelle 1320 zum Erzeugen eines Lichts in einem nicht-sichtbaren bzw. für den Menschen unsichtbaren Wellenlängenbereich und ein lichtempfindliches Element 1330 zum Empfangen eines beispielsweise in einem Finger reflektierten und remittierten Anteils des Lichts der ersten und zweiten Lichtquelle 1310, 1320 bzw. allgemein zum Empfangen eines Lichts in dem Wellenlängenbereich, in dem die erste Lichtquelle 1310 das sichtbare Licht erzeugt und die zweite Lichtquelle das nicht-sichtbare Licht erzeugt.

Die Steuereinrichtung 1410 ist ausgebildet, um die zweite Lichtquelle 1320 in zeitlichen Abständen anzuschalten, eine Auswertung des von dem lichtempfindlichen Element empfangenen Lichts in dem unsichtbaren Wellenlängenbereich der zweiten Lichtquelle dahin gehend durchzuführen, ob ein Finger an der optoelektronischen Sensoranordnung 1130 anliegt, und die erste Lichtquelle 1310 anzuschalten, sobald die Auswertung ergibt, dass ein Finger an der optoelektronischen Sensoranordnung 1130 anliegt.

Dabei können die zeitlichen Abstände, in denen die zweite Lichtquelle angeschaltet wird, periodisch sein, periodisch mit einer festen Periode oder einer abnehmenden Periodendauer sein, wobei die Periodendauer mit der Zeit, die seit einer letzten Erfassung verstrichen ist, abnimmt, oder beliebige andere zeitliche Abstände aufweisen.

Weitere Ausführungsbeispiele der Steuereinrichtung 1410 sind ausgebildet, um die erste Lichtquelle abzuschalten, sobald die zuvor genannte Auswertung ergibt, dass kein Finger an der optoelektronischen Sensoranordnung 1130 anliegt.

Alle Ausführungsbeispiele der Steuereinrichtung 1410 sind ausgebildet, die vorgenannte Auswertung über einen Zeitlauf des von dem lichtempfindlichen Element 1130 empfangenen Lichts in dem nicht-sichtbaren Wellenlängenbereich der zweiten Lichtquelle durchzuführen, um einen Pulsparameter, z. B. eine Pulswelle, zu erkennen, wenn ein Finger an der optoelektronischen Sensoranordnung 1130 anliegt, und entsprechend die erste Lichtquelle anzuschalten, wenn der Pulsparameter erkannt wird.

Aus dem zeitlichen Verlauf des von dem lichtempfindlichen Element empfangenen Lichts bzw. abhängig davon erzeugten Signals 1132 ist die Steuereinrichtung 1410 beispielsweise ausgebildet, die Pulswelle selbst zu erkennen, ihre spezielle Form zu erkennen (mittels entsprechender Auswertealgorithmen), die Frequenz und/oder Pulsamplitude des Pulses zu erkennen bzw. allgemein das Vorliegen eines Signals zu erkennen, das auf ein Vorhandensein eines Pulses und damit auf ein Anliegen eines Fingers oder ähnlichem auf der Sensoranordnung 1130 schließen lässt.

Lediglich beispielhaft für derartige Auswertealgorithmen seien hier Folgende genannt: a) Erkennen eines Pulses bzw. Anlegen eines Fingers, wenn das Signal 1132 eine bestimmte Anzahl von Nulldurchgängen pro Zeiteinheit aufweist, b) Erkennen eines Minimums bei Unterschreiten eines Minimumschwellwertes und eines Maximum bei Überschreiten eines Maximumschwellwertes, wobei die zeitliche Distanz zwischen dem Minimum und dem Maximum unter einem zeitlichen Schwellwert liegt, c) Erkennen einer Anstiegssteilheit, also einer maximalen Zunahmegeschwindigkeit der Signalamplitude, des vorab Tiefpass-gefilterten Signals anhand der Erkennung des Maximums der ersten Ableitung des vorab Tiefpass-gefilterten Zeitsignals, d) Detektion der signifikanten Signalstrukturen mit bekannter Breite mit Hilfe so genannter Öffnungsoperatoren (englisch "opening operator") und Schließungsoperatoren (englisch "closing operator") einer morphologischen Analyse des Zeitsignals, und e) Analyse der Spektraldichteschätzung, also einer Gewichtung der Frequenzteile.

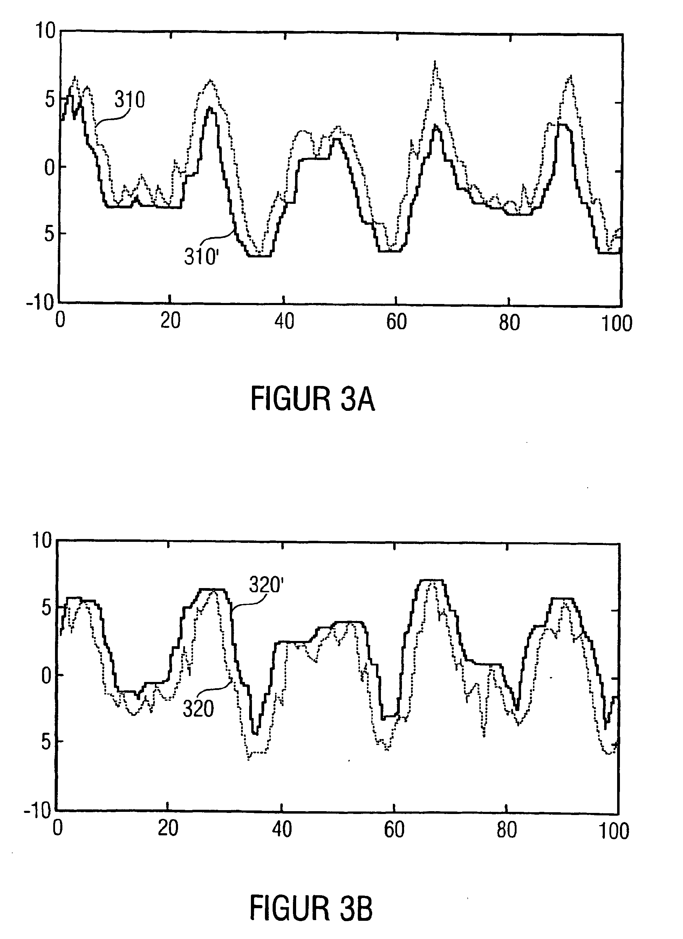

Im Folgenden wird die Funktionsweise von Ausführungsbeispielen eines Algorithmus zur Detektion der Pulswelle basierend auf einer Reihe morphologischer, nicht linearer Filteroperatoren, die vor allem in der Bildverarbeitung eingesetzt werden, beschrieben. Bei den Filteroperatoren handelt es sich um die so genannte "Erosion" (siehe Fig. 3A), die "Dilatation" (siehe Fig. 3B) und die sich daraus ergebende "opening" und "closing", auch "Öffnungsoperation" bzw. "morphologische Schließung" genannt.

Der Erosionsoperator ermittelt für jeden Wert eines digitalen Signals f 310 das Minimum der umliegenden M Signalwerte und weist dieses dann als neuen Wert zu. Dadurch werden positive Signalbereiche abgetragen und negative Signalbereiche ausgeweitet. Fig. 3A zeigt den Verlauf des Originalsignals 310 und des daraus erzeugten Signals 310' nach Erosion bzw. nach Durchführung der Erosionsoperation.

Der Dilatationsoperator ermittelt für jeden Wert eines digitalen Signals f 320 das Maximum der umliegenden M Signalwerte und weist dieses dann als neuen Wert zu. Dadurch werden positive Signalbereiche ausgeweitet und negative Signalbereiche abgetragen. Fig. 3B zeigt ein Beispiel eines Verlaufs eines Originalsignals 320 und eines daraus erzeugten Signals nach der Dilatation bzw. nach Durchführung der Dilatationsoperation.

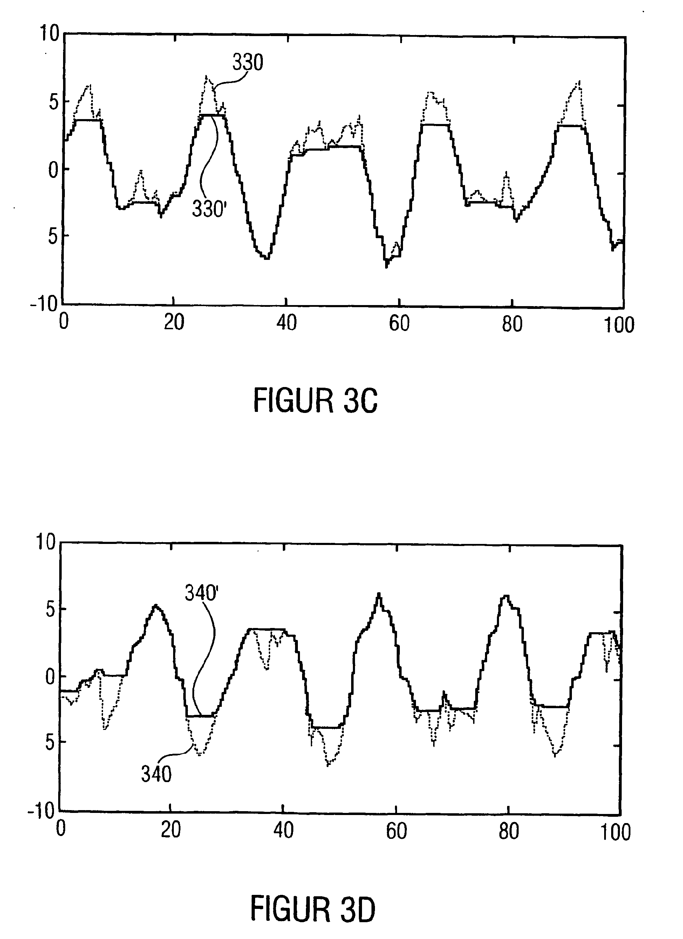

Der Opening-Operator ist definiert als Erosion mit nachfolgender Dilatation. Beide Operatoren zusammen bewirken in dieser Reihenfolge die Entfernung aller positiven Signalzacken, die im Englischen auch als "peaks" bezeichnet werden, innerhalb eines Intervalls von 0 bis M. Fig. 3C zeigt einen Verlauf eines Originalsignals 330 und das daraus erzeugten Signals 330' nach Durchführung der Opening-Operation.

Der Closing-Operator ist definiert als Dilatation mit nachfolgender Erosion. Beide Operatoren zusammen bewirken in dieser Reihenfolge die Entfernung aller negativen Signalzacken, die im Englischen als auch "pits" bezeichnet werden, innerhalb eines Intervalls von 0 bis M. Fig. 3D zeigt den Verlauf eines Originalsignals 340 und des durch die Anwendung der Closing-Operation auf das Ausgangssignal 340 erzeugte Signal 340' nach dem "closing".

Aufgrund ihrer Wirkungsweise können mit Hilfe der Opening- und Closing-Operatoren zum einen hochfrequentes Rauschen in Form von Spitzen, Zacken oder Ähnlichem aus einem Eingangssignal zu entfernt und zum anderen signifikante Signalstrukturen mit bekannter Breite, wie z.B. QRS-Komplexe in einem Elektrokardiogramm oder Ähnliches, detektiert werden.

Die vorliegende Erfindung schafft ferner ein Verfahren zum Erfassen eines Vitalparameters mittels einer optoelektronischen Sensoranordnung 1130, wie sie zuvor, z. B. anhand von Fig. 1, beschrieben wurde. Das Verfahren weist dabei die im Folgenden erläuterten Schritte auf.

Einschalten der zweiten Lichtquelle 1320 in zeitlichen Abständen.

Auswerten des von dem lichtempfindlichen Element 1330 empfangenen Lichts in dem unsichtbaren Wellenlängenbereich der zweiten Lichtquelle 1320 dahin gehend, ob ein Finger an der optoelektronischen Sensoranordnung 1130 anliegt, und Anschalten der ersten Lichtquelle, sobald die Auswertung ergibt, dass ein Finger an der optoelektronischen Sensoranordnung 1130 anliegt.

Für das Verfahren gelten die Ausführungen zur Vorrichtung zum Erfassen eines Vitalparameters entsprechend.

Als ein weiteres Ausführungsbeispiel des Verfahrens zum Erfassen eines Vitalparameters wird im Folgenden kurz eine Realisierung beschrieben, bei der der Lichtremissions-Sensor bzw. die optoelektronische Sensoranordnung 1130 in einem Schaltknauf eines Kraftfahrzeugs integriert ist. Dabei wird die infrarote Diode 1320 gleichzeitig mit dem Sensor 1130 eingeschaltet. Das Signal von der Messphotodiode 1330 wird dauernd abgefragt bzw. abgetastet und ausgewertet, um feststellen zu können, ob der Finger des Fahrers gegenüber dem optischen Sensor 1130 platziert ist und die Pulswellen detektierbar sind. Sobald eine Pulswelle detektiert wird, wird die rote Diode zusätzlich eingeschaltet und die Standardroutine für die pulsoxymetrische Messung aufgerufen. Zu dieser Zeit ist der Finger gegenüber dem optischen Sensor 1130 platziert und das rote Licht der roten Diode wird abgeschirmt. Sobald der Fahrer seinen Finger von dem optischen Sensor 1130 wieder wegnimmt, wird das als Verlust des optischen Kontakts zwischen Finger und Sensor 1130 interpretiert und die rote Diode 1310 wird wieder abgeschaltet. Der Algorithmus setzt weiter die Polling-Routine bzw. Abfrage-Routine am infraroten Kanal fort.

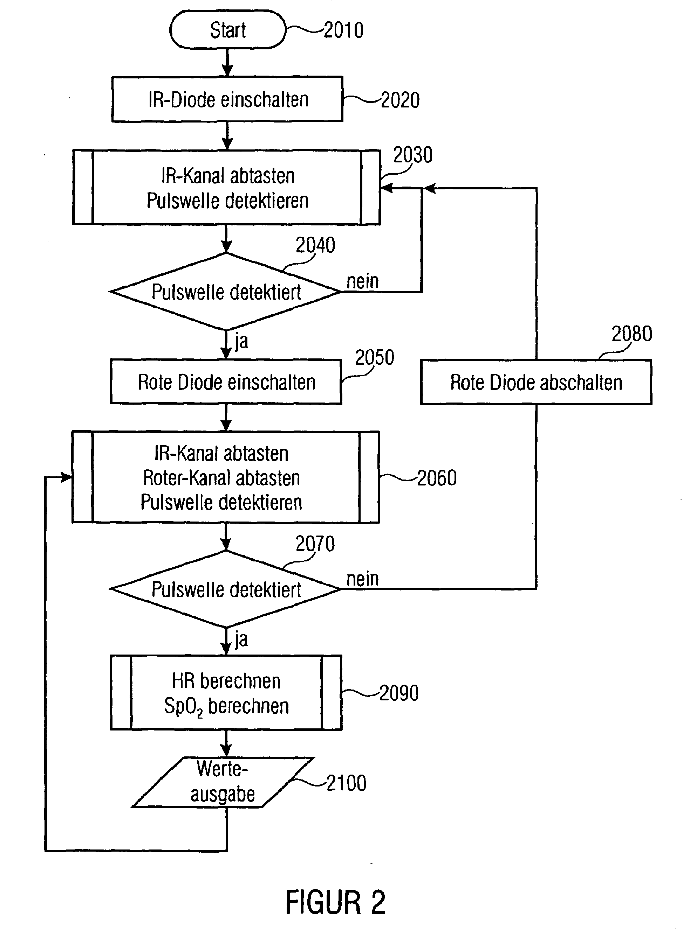

Im Folgenden wird ein Ausführungsbeispiel dieses Verfahrens zum Erfassen eines Vitalparameters anhand des in Fig. 2 gezeigten Flussdiagramms im Detail beschrieben.

Im Schritt 2010 wird die Vorrichtung 1100 bzw. das Verfahren zum Erfassen eines Vitalparameters gestartet und im Schritt 2020 die Infrarotdiode (IR-Diode) zusammen mit der optoelektronischen Sensoranordnung 1130 eingeschaltet. Im Schritt 2030 wird der Infrarot-Kanal (IR-Kanal) bzw. das Signal 1132 des lichtempfindlichen Elements abgetastet und, wie zuvor beschrieben, ausgewertet, um beispielsweise eine Pulswelle als Hinweis für das Anliegen eines Fingers zu detektieren. Im Schritt 2040 wird geprüft, ob eine Pulswelle detektiert wurde. Wenn keine Pulswelle detektiert wurde (nein), wird Schritt 2030 wiederholt. Wird eine Pulswelle detektiert (ja), wird im Schritt 2050 die rote Diode 1310 eingeschaltet und im Schritt 2060 der infrarote Kanal (IR-Kanal) und der rote Kanal abgetastet. Ferner wird in Schritt 2060 geprüft, ob weiterhin eine Pulswelle detektiert wird (Schritt 2070). Wird keine Pulswelle detektiert (nein), wird im Schritt 2080 die rote Diode 1310 wieder abgeschaltet und mit Schritt 2030 fortgefahren. Wird weiterhin eine Pulswelle detektiert (ja), wird die Standardroutine für die pulsoxymetrische Messung im Schritt 2090 durchgeführt. Hier wird beispielsweise basierend auf dem abgetasteten infraroten und roten Licht bzw. dem daraus resultierenden Signal 1132 des lichtempfindlichen Elements die Pulsrate bzw. Herzrate HR und/oder die Sauerstoffsättigung SpO₂ des Blutes berechnet, also in anderen Worten die Standardroutine zur Messung des einen oder mehrerer Vitalparameter durchgeführt. In Schritt 2100 werden diese Werte bzw. Vitalparameter dann ausgegeben, beispielsweise an ein Fahrerinformationssystem oder an einen Ärzte-PC übertragen. Nach Schritt 2100 wird mit dem Schritt 2060 weiter fortgefahren und dieser solange wiederholt, bis keine Pulswelle detektiert wird.

Bezug nehmend auf die vorhergehenden Erläuterungen schaffen Ausführungsbeispiele auch eine "Einschaltvorrichtung eines optischen Sensors zur Detektion einer Pulswelle des Fahrers im Schaltknauf" und/oder ein "Verfahren zur Steuerung eines optischen Sensors zur Detektion einer Pulswelle des Fahrers im Schaltknauf".

Dabei wird der Betrieb des Sensors optisch nicht sichtbar bzw. unsichtbar für den Anwender bzw. Fahrer gemacht.

Abhängig von den Gegebenheiten können die Ausführungsbeispiele der erfindungsgemäßen Verfahren in Hardware oder in Software implementiert werden. Die Implementierung kann auf einem digitalen Speichermedium, insbesondere einer Diskette, CD oder DVD mit elektronisch auslesbaren Steuersignalen erfolgen, die so mit einem programmierbaren Computersystem zusammenwirken, dass eines der Ausführungsbeispiele der erfindungsgemäßen Verfahren ausgeführt wird. Allgemein bestehen die Ausführungsbeispiele der vorliegenden Erfindung somit auch in Software-Programm-Produkten bzw. Computer-Programm-Produkten bzw. Programmprodukten mit einem auf einem maschinenlesbaren Träger gespeicherten Programmcode zur Durchführung eines Ausführungsbeispiels der erfindungsgemäßen Verfahren, wenn eines der Software-Programm-Produkte auf einem Rechner oder auf einen Prozessor abläuft. In anderen Worten ausgedrückt, kann ein Ausführungsbeispiel der vorliegenden Erfindung somit als ein Computerprogramm bzw. Software-Programm bzw. Programm mit einem Programmcode zur Durchführung eines Ausführungsbeispiels eines erfindungsgemäßen Verfahrens realisiert werden, wenn das Programm auf einem Prozessor abläuft. Dabei kann der Prozessor dabei von einem Computer, einer Chipkarte, einem digitalen Signalprozessor oder einem anderen integrierten Schaltkreis gebildet sein.

## Patentansprüche

1. Vorrichtung zum Erfassen eines Vitalparameters mit folgenden Merkmalen:
einer optoelektronischen Sensoranordnung (1130) zum Erfassen des Vitalparameters mittels Lichtremission an einem Finger, wobei die optoelektronische Sensoranordnung eine erste Lichtquelle (1310) zum Erzeugen eines Lichts im sichtbaren Wellenlängenbereich aufweist, eine zweite Lichtquelle (1320) zum Erzeugen eines Lichts in einem nicht-sichtbaren Wellenlängenbereich aufweist und ein lichtempfindliches Element (1330); und
einer Steuereinrichtung (1410), die ausgebildet ist, um die zweite Lichtquelle (1320) in zeitlichen Abständen anzuschalten, eine Auswertung des von dem lichtempfindlichen Element empfangenen Lichts in dem unsichtbaren Wellenlängenbereich der zweiten Lichtquelle dahin gehend durchzuführen, ob ein Finger an der optoelektronischen Sensoranordnung (1130) anliegt, und die erste Lichtquelle (1310) anzuschalten, sobald die Auswertung ergibt, dass ein Finger an der optoelektronischen Sensoranordnung anliegt,
**gekennzeichnet dadurch, dass**
die Steuereinrichtung (1410) ausgebildet ist, die Auswertung über einen Zeitverlauf des von dem lichtempfindlichen Element (1330) empfangenen Lichts in dem unsichtbaren Wellenlängenbereich der zweiten Lichtquelle (1320) durchzuführen, um einen Pulsparameter zu erkennen, wenn ein Finger an der optoelektronischen Sensoranordnung (1130) anliegt, und die erste Lichtquelle (1310) anzuschalten, wenn eine Pulswelle erkannt wird.

2. Vorrichtung nach Anspruch 1, wobei die Steuereinrichtung ausgebildet ist, um die erste Lichtquelle (1310) abzuschalten, sobald die Auswertung ergibt, dass kein Finger an der optoelektronischen Sensoranordnung anliegt.

3. Vorrichtung nach Anspruch 1 oder 2, wobei das von dem lichtempfindlichen Element empfangene Licht ein von dem anliegenden Finger remittierter Anteil des von der zweiten Lichtquelle (1320) erzeugten Lichts ist.

4. Vorrichtung nach einem der Ansprüche 1 bis 3, wobei der Pulsparameter eine Pulswelle, Pulsfrequenz oder Pulsamplitude ist.

5. Vorrichtung nach einem der Ansprüche 1 bis 4, wobei der Vitalparameter eine Sauerstoffsättigung des Bluts ist.

6. Verfahren zum Erfassen eines Vitalparameters mittels einer optoelektronischen Sensoranordnung (1130) zum Erfassen des Vitalparameters mittels Lichtremission, wobei die optoelektronische Sensoranordnung eine erste Lichtquelle (1310) zum Erzeugen eines Lichts im sichtbaren Wellenlängenbereich aufweist, eine zweite Lichtquelle (1320) zum Erzeugen eines Lichts in einem nicht-sichtbaren Wellenlängenbereich aufweist und ein lichtempfindliches Element (1330) aufweist, und wobei das Verfahren folgende Schritte aufweist:
Einschalten der zweiten Lichtquelle (1320) in zeitlichen Abständen;
Auswerten des von dem lichtempfindlichen Element (1330) empfangenen Lichts in dem unsichtbaren Wellenlängenbereich der zweiten Lichtquelle (1320) dahin gehend, ob ein Finger an der optoelektronischen Sensoranordnung anliegt; und
Anschalten der ersten Lichtquelle, sobald die Auswertung ergibt, dass ein Finger an der optoelektronischen Sensoranordnung anliegt,
**gekennzeichnet dadurch, dass**
das Auswerten über einen Zeitverlauf des von dem lichtempfindlichen Element (1330) empfangenen Lichts in dem unsichtbaren Wellenlängenbereich der zweiten Lichtquelle (1320) durchgeführt wird, um einen Pulsparameter zu erkennen, wenn ein Finger an der optoelektronischen Sensoranordnung (1130) anliegt, und die erste Lichtquelle (1310) angeschaltet wird, wenn eine Pulswelle erkannt wird.

7. Verfahren nach Anspruch 6 mit folgendem Schritt:
Abschalten der ersten Lichtquelle (1310), sobald die Auswertung ergibt, dass kein Finger an der optoelektronischen Sensoranordnung anliegt.

## Claims

1. A device for detecting a vital parameter, comprising:
an optoelectronic sensor arrangement (1130) for detecting the vital parameter by means of light remission at a finger, wherein the optoelectronic sensor arrangement comprises a first light source (1310) for generating light in a visible wavelength range, a second light source (1320) for generating light in a non-visible wavelength range and a light sensitive element (1330); and
a controller (1410) which is implemented to switch on the second light source (1320) in temporal intervals, execute an evaluation of the light received from the light-sensitive element in the invisible wavelength range of the second light source with respect to whether a finger is applied to the optoelectronic sensor arrangement (1130) and to switch on a first light source (1310) as soon as the evaluation indicates that a finger is applied to the optoelectronic sensor arrangement,
**characterized in that**
the controller (1410) is implemented to execute the evaluation over a temporal course of the light received from the light sensitive element (1330) in the invisible wavelength range of the second light source (1320) to detect a pulse parameter when a finger is applied to the optoelectronic sensor arrangement (1130) and to switch on the first light source (1310) when a pulse wave is detected.

2. The device according to claim 1, wherein the controller is implemented to switch off the first light source (1310) as soon as the evaluation indicates that no finger is applied to the optoelectronic sensor arrangement.

3. The device according to claim 1 or 2, wherein the light received from the light-sensitive element is a portion of the light generated by the second light source (1320) remitted by the applied finger.

4. The device according to one of claims 1 to 3, wherein the pulse parameter is a pulse wave, a pulse frequency or a pulse amplitude.

5. The device according to one of claims 1 to 4, wherein the vital parameter is an oxygen saturation of the blood.

6. A method for detecting a vital parameter by means of an optoelectronic sensor arrangement (1130) for detecting the vital parameter by means of light remission, wherein the optoelectronic sensor arrangement comprises a first light source (1310) for generating light in the visible wavelength range, a second light source (1320) for generating light in a non-visible wavelength range and a light sensitive element (1330) , and wherein the method further comprises:
switching on the second light source (1320) in temporal intervals;
evaluating the light received from the light-sensitive element (1330) in the invisible wavelength range of the second light source (1320) with respect to whether a finger is applied to the optoelectronic sensor arrangement; and
switching on the first light source as soon as the evaluation indicates that a finger is applied to the optoelectronic sensor arrangement,
**characterized in that**
executing is performed over a temporal course of the light received from the light sensitive element (1330) in the invisible wavelength range of the second light source (1320) to detect a pulse parameter when a finger is applied to the optoelectronic sensor arrangement (1130) and to switch on the first light source (1310) when a pulse wave is detected.

7. The method according to claim 6, comprising:
switching off the first light source (1310) as soon as the evaluation indicates that no finger is applied to the optoelectronic sensor arrangement.

## Revendications

1. Dispositif pour détecter un paramètre vital, aux caractéristiques suivantes:
un aménagement de capteur optoélectronique (1130) destiné à détecter le paramètre vital au moyen d'une rémission de lumière à un doigt, l'aménagement de capteur optoélectronique présentant une première source de lumière (1310) destinée à générer une lumière dans la plage de longueurs d'onde visibles, une deuxième source de lumière (1320) destinée à générer une lumière dans une plage de longueurs d'onde non visibles et un élément sensible à la lumière (1330); et
un moyen de commande (1410) qui est réalisé pour enclencher la deuxième source de lumière (1320) à des intervalles de temps, réaliser une évaluation de la lumière dans la plage de longueurs d'onde invisibles reçue de la deuxième source de lumière par l'élément sensible à la lumière, pour savoir si un doigt est appliqué sur l'aménagement de capteur optoélectronique (1130), et enclencher la première source de lumière (1310) dès que l'évaluation indique qu'un doigt est appliqué sur l'aménagement de capteur optoélectronique,
**caractérisé par le fait que**
le moyen de commande (1410) est réalisé pour effectuer l'évaluation sur une durée de temps de la lumière dans la plage de longueurs d'onde invisibles reçue de la deuxième source de lumière (1320) par l'élément sensible à la lumière (1330), pour identifier un paramètre de pulsation lorsqu'un doigt est appliqué sur l'aménagement de capteur optoélectronique (1130), et enclencher la première source de lumière (1310) lorsqu'une onde d'impulsion est identifiée.

2. Dispositif selon la revendication 1, dans lequel le moyen de commande est réalisé pour déclencher la première source de lumière (1310) dès que l'évaluation indique qu'aucun doigt n'est appliqué sur l'aménagement de capteur optoélectronique.

3. Dispositif selon la revendication 1 ou 2, dans lequel la lumière reçue par l'élément sensible à la lumière est une part réémise par le doigt appliqué de la lumière générée par la deuxième source de lumière (1320).

4. Dispositif selon l'une des revendications 1 à 3, dans lequel le paramètre de pulsation est une onde de pulsation, une fréquence de pulsation ou une amplitude de pulsation.

5. Dispositif selon l'une des revendications 1 à 4, dans lequel le paramètre vital est une saturation d'oxygène du sang.

6. Dispositif pour détecter un paramètre vital au moyen d'un aménagement de capteur optoélectronique (1130) pour détecter le paramètre vital au moyen d'une rémission de lumière, l'aménagement de capteur optoélectronique présentant une première source de lumière (1310) destinée à générer une lumière dans la plage de longueurs d'onde visibles, une deuxième source de lumière (1320) destinée à générer une lumière dans une plage de longueurs d'onde non visibles et un élément sensible à la lumière (1330), et dans lequel le procédé présente les étapes suivantes consistant à:
enclencher la deuxième source de lumière (1320) à des intervalles de temps;
évaluer la lumière dans la plage de longueurs d'onde invisibles de la deuxième source de lumière (1320) reçue par l'élément sensible à la lumière (1330) pour savoir si un doigt est appliqué sur l'aménagement de capteur optoélectronique; et
enclencher la première source de lumière dès que l'évaluation indique qu'un doigt est appliqué sur l'aménagement de capteur optoélectronique,
**caractérisé par le fait que**
l'évaluation est réalisée sur une durée de temps de la lumière dans la plage de longueurs d'onde invisibles de la deuxième source de lumière (1320) reçue par l'élément sensible à la lumière (1330), pour identifier un paramètre de pulsation lorsqu'un doigt est appliqué sur l'aménagement de capteur optoélectronique (1130), et la première source de lumière (1310) est enclenchée lorsqu'une onde de pulsation est identifiée.

7. Procédé selon la revendication 6, à l'étape suivante consistant à:
déclencher la première source de lumière (1310) dès que l'évaluation indique qu'aucun doigt n'est appliqué sur l'aménagement de capteur optoélectronique.
